# EUROPÄISCHE PATENTSCHRIFT

(11) **EP 1 223 899 B1**
(45) Veröffentlichungstag und Bekanntmachung des Hinweises auf die Patenterteilung: **28.07.2004**
(21) Anmeldenummer: 00971109.4
(22) Anmeldetag: 24.10.2000
(51) Int. Cl.: A61F 5/058, A61F 5/055

(54) **EINRICHTUNG ZUR STÜTZUNG UND STABILISIERUNG EINES VERLETZTEN**
DEVICE FOR SUPPORTING AND STABILIZING AN INJURED PERSON
DISPOSITIF SERVANT A SUPPORTER ET A STABILISER UN BLESSE

(30) Priorität: 25.10.1999 AT 179699
(43) Veröffentlichungstag der Anmeldung: 24.07.2002
(73) Patentinhaber: Kohlbrat & Bunz Gesellschaft m.b.H, 5550 Radstadt (AT)
(72) Erfinder: RUGFELT, Hakan, SE-239 41 Falsterbo (SE); RENBERG, Bo, SE-693 35 Degerfors (SE)
(74) Vertreter: Torggler, Paul Norbert, Dr.
(86) Internationale Anmeldenummer: PCT/AT2000/000276
(87) Internationale Veröffentlichungsnummer: WO 2001/030280

(56) Entgegenhaltungen:
- FR-A- 2 530 946
- FR-A- 2 638 965
- US-A- 3 745 998
- US-A- 4 261 349
- US-A- 5 718 669

## Beschreibung

Die Erfindung betrifft eine Einrichtung zur Stützung und Stabilisierung eines Verletzten bzw. eines verletzten Körperteils, mit einem flexiblen, um den Körperteil festlegbaren Folienelement, dessen beiden Folien einen luftdichten, evakuierbaren Innenraum einschließen, in dem lose Partikel enhaltende langgestreckte Kammern vorgesehen sind.

Als Vakuummatratzen, Vakuumwesten und Vakuumschienen bezeichnete Rettungs- und Transporteinrichtungen weisen eine Hülle aus einer luftdichten Kunststoffolie und eine Füllung aus einem Kunststoffgranulat, insbesondere aus geschäumten Polystyrolkugeln, auf und können nach der Anpassung und Fixierung an einem ruhig zu stellenden Körperteil sind mittels einer Saugpumpe evakuiert werden. Dies führt zu einer dichten Packung des eingefüllten Granulats und somit zu einer Versteifung des flexiblen Elements, das auf diese Weise eine im wesentlichen starre Hülle oder Manschette des Körperteils bildet. Für eine möglichst universelle Fixierung am Körperteil sind die Befestigungsgurte in Umfangsrichtung des Körperteils angeordnet und mit üblichen einstellbaren Verbindungsbeschlägen versehen. Vakuummatratzen und -schienen sind hauptsächlich für eine horizontale Einsatzlage geeignet. Bei Verwendung in einer nicht horizontalen, beispielsweise bei sitzenden Verletzten, besteht die Gefahr, daß die Füllung in einem höheren Bereich eine dünnere Schicht bildet oder sogar fehlt, weil sie sich während der Handhabung von oben nach unten verlagert hat. Es wurde daher vorgeschlagen (US 5,154,185 A) den Innenraum durch horizontale Trennwände in schmälere Kammern zu unterteilen, wobei die Trennwände Durchbrechungen aufweisen, die gerade so groß sind, daß Teile der Füllung nur händisch hindurchgedrückt werden können. Die horizontalen Trennwände sind an der patientenseitigen Folie direkt und an der Außenseite an einer inneren Schicht befestigt, die ihrerseits mit der äußeren Folie verbunden ist. Die beiden Folien aus einem luftdichten Material sind entlang ihrer Ränder miteinander verbunden. Die Herstellung einer derartigen Einrichtung ist daher verhältnismäßig schwierig, da zumindest an einer Seite die Befestigung der Trennwände vom Inneren der Kammern heraus erfolgen muß.

Das Stützelement der US 5,154,185 A ist auch auf einem Sitz fixierbar, wobei im Oberkörperbereich versteifende Längsstege eingesetzt werden. Eine spezielle Kopf- und Nackenstütze ist nicht vorgesehen.

Aus der WO 99/16392 A ist eine Einrichtung der eingangs genannten Art bekannt, die ebenfalls insbesondere für einen aufrechten Transport eines Verletzten geeignet ist, wobei ein evakuierbares Oberkörperstützkissen und ein getrennt davon evakuierbares Kopfstützkissen an einem versteifenden Rahmen befestigt sind, wobei das Kopfstützkissen beiseitig mit einer Schulterabstützung versehen ist. Die Kissen bestehen aus zwei luftdichten Folien, die entlang ihrer Ränder und an einigen Stellen im Inneren miteinander verbunden sind und einen evakuierbaren Innenraum einschließen, in dem lose Partkel enthalten sind. Die inneren Verbindungen behindern die freie Bewegung des Granulats, sodaß eine etwa gleichmäßige Verteilung trotz der aufrechten Verwendung im wesentlichen erhalten bleibt. Auch die Herstellung dieser Stützeinrichtung ist verhältnismäßig kompliziert.

Die Erfindung hat es sich nun zur Aufgabe gestellt, eine einfacher herzustellende Einrichtung zur Stützung und Stabilisierung eines verletzten Körperteils zu schaffen und erreicht dies dadurch, daß der Innenraum durch mindestens zwei luftdurchlässige, flexible Trennwände in zumindest drei Fächer unterteilt ist, und die Kammern in zumindest einem der drei Fächer ausgebildet sind und sich in Umfangsrichtung des zu stabilisierenden Körperteils erstrecken.

Die Unterteilung des Innenraumes parallel zu den Folien in drei Fächer, also flache Teilräume, ermöglicht es, die Einrichtung in Teilschichten so zu fertigen, daß die Verbindung der einzelnen Elemente an gut bzw. leicht zugänglichen Stellen vorgesehen und ausgeführt werden können. Aufbauend auf diesen Grundmerkmalen lassen sich verschiedene bevorzugte Ausführungen festlegen.

So weist eine erste Ausführung nur drei Fächer auf, wobei die Kammern im mittleren Fach vorgesehen sind und durch Stege zwischen den flexiblen Trennwänden gebildet sind. Die Stege sind schräggestellt, sodaß sich die Kammern und die darin eingefüllten Partikel überlappen.

Eine zweite Ausführung eines derartigen Innenelementes sieht vor, daß die schräggestellten Stege durch eine in Wellen gelegte Einlage gebildet sind, die jeweils in den Umkehrbereichen mit den beiden flexiblen Trennwänden verbunden sind.

Die beiden die Kammem einschließenden Trennwände stellen daher jeweils ein getrennt herstellbares Innenelement dar, das mit den Partikeln gefüllt und entlang seines Umfangs im Randbereich einer Folie befestigt wird. Anschließend wird die zweite Folie mit der ersten verbunden. Das vorgefertigte, einen Einsatzkörper bildende Innenelement kann aber auch zwischen die Ränder der beiden Folien eingesetzt und gemeinsam mit diesen verbunden werden. Der Einsatzkörper kann beispielsweise aus mehreren etwa Z-förmig vernähten Streifen hergestellt werden. Die beiden äußeren Fächer sind leer, sodaß sich beim Absaugen der Luft die Trennwände von innen an die Folien anlegen.

In einer weiteren Ausführung ist der Innenraum ebenfalls in drei Fächer unterteilt, wobei die Kammern aber in den beiden äußeren Fächern vorgesehen sind und das mittlere Fach leer ist. In dieser Ausführung werden jeweils eine Trennwand und eine Folie durch mehrere parallele Verbindungsnähte in Kammern unterteilt, die Kammern mit Partikel gefüllt und anschließend die beiden Folien entlang ihrer Ränder verbunden. Gegenüberliegende Verbindungsnähte sind jeweils um die halbe Kammerbreite versetzt, sodaß die beiden Kammernreihen einander überlappen. Die Überlappung der Kammern führt trotz fehlender Partikel im Bereich der Verbindungsnähte zu einer annähernd gleichmäßigen Verteilung der Füllung.

In einer dritten Ausführung ist der Innenraum durch vier Trennwände in fünf Fächer unterteilt und die Kammern sind im zweiten und im vierten Fach vorgesehen. Hiefür sind die Trennwände paarweise miteinander durch parallele Verbindungsnähte verbunden, wobei auch in dieser Ausführung die gegenüberliegenden Verbindungsnähte jeweils um die halbe Kammerbreite versetzt sind, sodaß jeweils eine Verbindungsnaht im Bereich eines Kammerscheitels liegt, und die beiden Reihen von Kammern einander wiederum überlappen. Bei der Herstellung dieser Ausführung werden zuerst die beiden aus je zwei Trennwänden bestehenden Einsatzkörper hergestellt und mit den Partikeln gefüllt. Anschließend werden die Einsatzkörper jeweils entlang ihres Umfangs im Randbereich einer Folie befestigt und schließlich die beiden Folien entlang ihrer Ränder verbunden.

Da die Folien bevorzugt aus einem Polyurethan und die Trennwände und Stege durch Gewebebahnen aus Polypropylenfasem gewebt sind, sind die Folien miteinander bzw. mit den Trennwänden bevorzugt verschweißt, und die Trennwände sind miteinander bzw. mit den Stegen vernäht.

Wenn die erfindungsgemäße Einrichtung zur Stützung und Stabilisierung des gesamten Oberkörpers einen Rumpfteil und einen Kopfteil aufweist, so ist bevorzugt vorgesehen, daß die Partikel in Kammern vorgesehen sind, deren Breite im Kopfteil kleiner ist als im Rumpfteil.

Für die Schulterabstützung des Kopfteiles kann weiters vorgesehen sein, daß jede Schulterabstützung mit Verbindungsgurten zur Vorderseite des Rumpfteiles versehen ist. Die Schulterabstützung ist im flachen Zustand des Folienelements durch seitlich oberhalb des Rumpfteils vorstehende Laschen gebildet, die um den Kopf nach vorne gebogen werden, sodaß sie auf der Schulter aufliegen. Die Laschen sind insbesondere so groß, daß sie nach unten gebogen werden können und den Bereich der Schlüsselbeine überdecken. Die auf den Schultern aufliegenden Laschen ergeben nach der Evakuierung eine stabile Auflage des Kopfteiles auf der Schulter des durch den Rumpfteil immobilisierten Verletzten, d.h. es ergibt sich ohne versteifende Rahmen- oder Einlagenteile eine hervorragende Kopfabstützung.

Die Einrichtung zur Stützung und Stabilisierung eines verletzten Körperteils ist üblicherweise mit Befestigungsgurten versehen, die in Umfangsrichtung des Körperteils geschlossen werden können.

Eine für die Stabilisierung eines sitzenden Verletzten vorteilhafte Eigensteife in vertikaler Richtung kann insbesondere durch Aufbringung eines weiteren Folienstückes auf der Außenseite der vom Verletzten abgewandten Folie erreicht werden, das beispielsweise durch einander kreuzende Verbindungsnähte an der Folie befestigt ist. Die Befestigungsgurte sind dann bevorzugt zumindest teilweise zwischen der vom verletzten Körperteil abgewandten Folie und dem zusätzlichen Folienstück angeordnet.

Bevorzugt überlagern die Kammern einander teilweise so, daß bei evakuiertem Innenraum Partikel benachbarter Kammern einander zu einer im wesentlichen gleichmäßigen Schicht ergänzen.

Bevorzugte Ausführungen der Einrichtung bilden eine Weste für die Oberkörperstabilisierung bzw. eine Halsmanschette.

Nachstehend wird nun die Erfindung anhand der Figuren der beiliegenden Zeichnung näher beschrieben, ohne darauf beschränkt zu sein. Es zeigen
Fig. 1 eine Draufsicht auf die am Patienten anliegende Seite einer Einrichtung für die Oberkörper- und Kopfstabilisierung,
Fig. 2 eine Draufsicht auf deren Außenseite,
Fig. 3 eine Vorderansicht eines stabilisierten Patienten,
Fig. 4 eine Seitenansicht des Patienten,
Fig. 5 eine schematische Ansicht der Kammernverteilung,
Fig. 6 ein Schnitt nach der Linie VI-VI der Fig. 5,
Fig. 7 eine Draufsicht auf die am Patienten anliegende Seite einer zweiten Ausführung einer Einrichtung für die Oberkörper- und Kopfstabilisierung,
Fig. 8 eine Draufsicht auf die Außenseite der zweiten Ausführung und
Fig. 9 bis 12 Schnitte nach der Linie X-X der Fig. 7 durch vier verschiedene Ausführungsbeispiele.

Eine Einrichtung zur Stabilisierung bzw. Stützung des Oberkörpers eines Verletzten weist ein Folienelement 1 mit einem Kopfteil 20 und mit einem durch Armausschnitte 21 getrennten Rumpfteil 22 auf.

Das Folienelement 1 ist aus zwei luftdichten Folien 2', 2" zusammengesetzt, die insbesondere aus einem Polyurethan bestehen, und längs ihrer Ränder 23 miteinander verschweißt sind. Die am Verletzten zur Anlage kommende Folie 2' ist glatt, und auf der Außenfolie 2" ist ein versteifendes, zusätzliches Folienstück 10 durch mehrere, einander kreuzende Längs- und Querschweißnähte fixiert. Das Folienstück 10 trägt Ein- und Austrittsschlitze 12 für Befestigungsgurte 11, die teilweise zwischen dem Folienstück 10 und der Außenfolie 2" verlaufen und an ihren nicht gezeigten Enden mit üblichen Verbindungsbeschlägen versehen sind. Mit Ausnahme unterer Gurten 11', die zur Fixierung an den Oberschenkeln dienen, erstrecken sich die Gurte 11 in Umfangsrichtung des zu umschließenden Oberkörpers. In der Außenfolie 2" ist ein Ventil 13 angeordnet, durch das Luft aus dem mit Partikeln 6, beispielsweise Kunststoffkugeln aus geschäumtem Polystyrol gefüllten Innenraum abgesaugt werden kann. Das vor der Anwendung weiche, flache Folienelement 1 ist vor allem zur Stabilisierung sitzender Verletzter geeignet und wird aus dem flachen Zustand um den Rücken des Verletzten gelegt und an den Seiten vorgezogen und mit Hilfe der Gurte am Kopf, Rumpf und Oberschenkeln fixiert. Wird nunmehr die Luft abgesaugt, so versteift sich das Folienelement in der an die Körperform angepaßten Gestalt, da den Partikeln 6 der Bewegungsraum entzogen ist und sie durch den äußeren Luftdruck gegeneinander gepreßt sind.

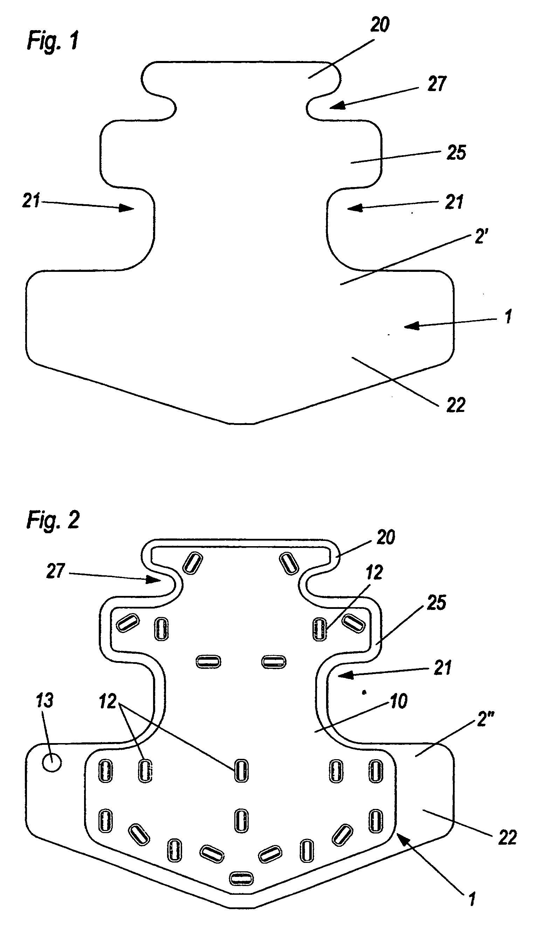

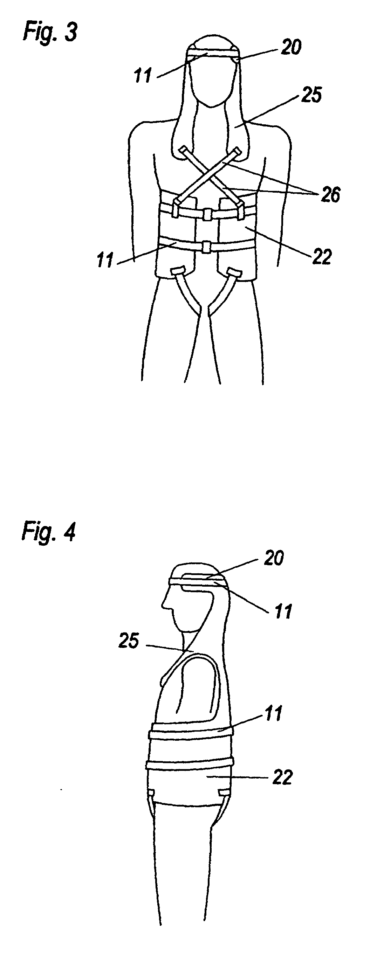

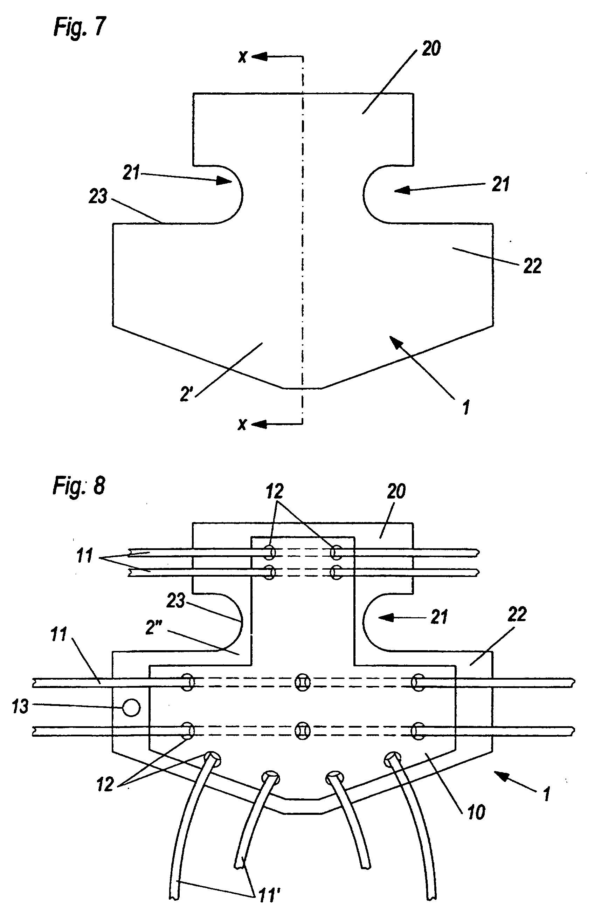

In der Ausführung nach Fig. 1 bis 6 weist das Folienelement 1 oberhalb des Armausschnittes 21 seitlich abstehende Laschen 25 auf, die um den Nackenbereich nach vorne und über die Schultern nach unten gelegt werden können, wobei sie etwas unterhalb des Schlüsselbeinbereiches enden. Schräg geführte bzw. gekreuzte Gurten 26 verbinden die Laschen 25 mit dem Rumpfteil 22 (Fig. 3,4). Zwischen den Laschen 25 und den Seitenenden des Kopfteiles 20 sind Einschnitte 27, die wie Fig. 4 zeigt, den mittleren Gesichtsbereich freilassen. Der Kopfteil 20 ist im Bereich der Schläfen und Stirn des Verletzten auf einen schmalen Streifen reduziert In der Ausführung nach Fig. 7 und 8 ist der Kopfteil 22 höher und reicht bis zum Armausschnitt 21, sodaß die Seitenbereiche direkt auf der Schulter aufliegen. Der übrige Aufbau entspricht dem unter Verweis auf die Fig. 1 und 2 beschriebenen Aufbau.

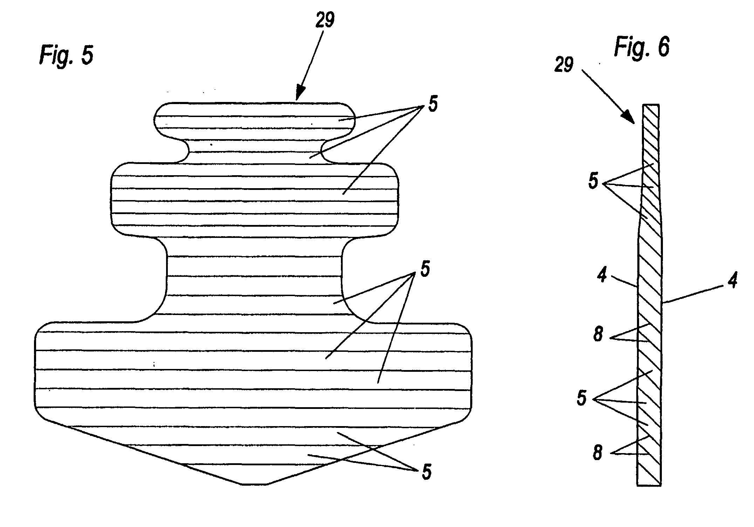

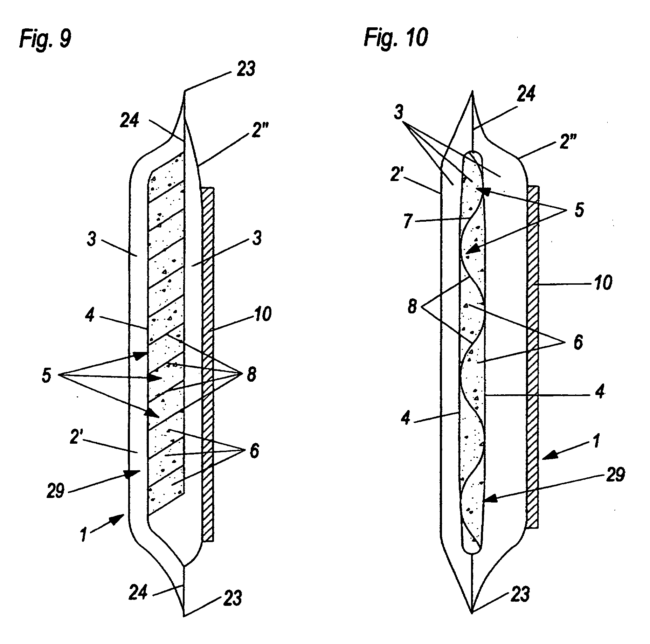

Um während des Anlegens am sitzenden Verletzten zu vermeiden, daß die Partikel 6 nach unten rutschen, sodaß in den oberen Bereichen, vor allem im Kopfteil 20 zu wenig Partikel für die gewünschte Versteifung vorhanden sind, ist der Innenraum des Folienelementes 1 in schmale Kammern 5 unterteilt, in denen jeweils die Partikel 6 angeordnet sind. Die Längserstreckung der schmalen Kammern 5 ist parallel zu den Gurten 11 und daher in Umfangsrichtung des zu stabilisierenden Körperteils, und daher in aufrechter Verwendungsposition horizontal, sodaß das Absinken der Füllung nach unten verhindert wird. Wie Fig. 5 und 6 zeigen, können obere Kammern 5 des Kopfteiles 20 schmäle als untere Kammern 5 des Rumpfteiles 22 sein. Um ein derartiges Folienelement 1 rationell und einfach herstellen zu können, sind verschiedene in Fig. 9 bis 12 dargestellte Möglichkeiten gegeben.

Fig. 9 zeigt eine Ausführung, in der der Innenraum des Folienelementes 1 durch zwei luftdurchlässige Trennwände 4 in drei Fächer 3 unterteilt ist. Die beiden äußeren Fächer 3 sind leer und beinhalten daher nur Luft. Das mittlere Fach ist durch schräggestellte Stege 8 in die erwähnten, mit Partikeln 6 gefüllten Kammern 5 unterteilt. Durch die Schrägstellung der Stege 8 wird eine annähernd gleichmäßig dicke Schicht der Partikel 6 über die gesamte Fläche des Folienelementes 1 erreicht. Die beiden Trennwände 4 und die Stege 8 stellen einen vorfertigbaren Einsatzkörper 29 dar, der nach Füllung mit den Partikeln 8 entlang seines Randes 24 mit der Folie 2' verbunden wird. Anschließend kann dann die zweite Folie 2" mit der mit dem Einsatzkörper versehenen Folie 2' verbunden werden.

Die Ausführung nach Fig. 10 ist ähnlich. Auch hier sind die Partikel 6 im mittleren Fach 3 vorgesehen, wobei die Kammern 5 durch eine die schräggestellten Stege 8 bildende, wellenförmige Einlage 7 getrennt sind, die alternierend an den beiden Trennwänden 4 fixiert ist. Auch hier stellen die beiden Trennwände 4 mit der wellenförmigen Einlage 7 einen vorgefertigten Einsatzkörper 29 dar, der entlang seines Randes 24 mit der Folie 2' verbunden wird, bevor die zweite Folie 2" angebracht wird.

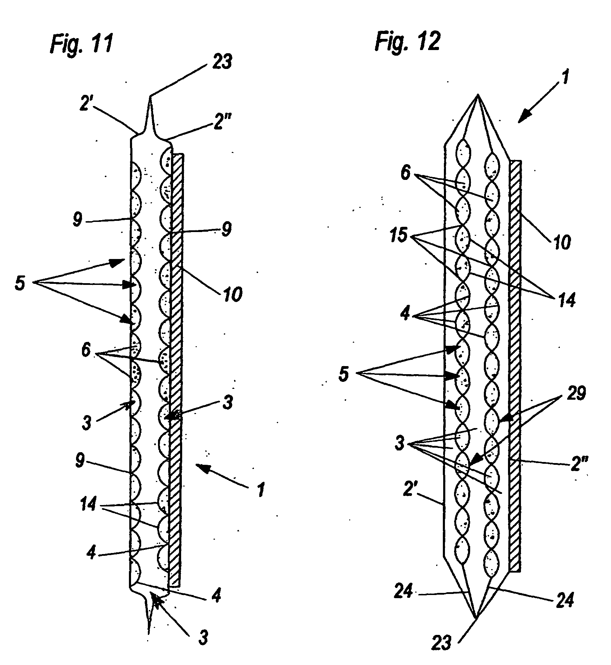

Fig. 11 zeigt eine weitere Ausführung, in der der Innenraum des Folienelementes 1 durch zwei Trennwände 4 in die Fächer 3 unterteilt ist, wobei aber die beiden äußeren Fächer 3 partikelgefüllte Kammern 5 enthalten, und das mittlere Fach 3 leer ist. Die Kammern 5 sind in dieser Ausführung durch zueinander parallele Verbindungsnähte 9 zwischen der luftdurchlässigen Trennwand 4 und der Folie 2' bzw. 2" gebildet. Um die Schicht der Partikel 6 über die gesamte Fläche des Folienelementes 1 etwa gleich dick zu erhalten, sind die gegenüberliegenden Verbindungsnähte 9 jeweils um die halbe Breite der Kammern 5 versetzt. Beim Absaugen der Luft aus dem Folienelement 1 legt sich daher jeweils ein Scheitel 14 einer Kammer 5 in den Bereich eins Verbindungsnaht 9 zu der gegenüberliegenden Seite. In dieser Ausführung werden zuerst die Innenseiten der Folien 2', 2" jeweils mit einer Trennwand 4 verbunden, und die Kammern 5 hergestellt und gefüllt. Anschließend werden wiederum die beiden Folien 2', 2" miteinander entlang ihrer Ränder 23 verschweißt.

Eine vierte Ausführung ist in Fig. 12 gezeigt. Hier ist der Innenraum durch vier Trennwände 4 in fünf Fächer 3 unterteilt, wobei im zweiten und vierten Fach 3 Kammern 5 ausgebildet sind und die beiden äußersten Fächer sowie das mittlere Fach leer bleiben, d. h. nur Luft enthalten. Hier sind die Kammern 5 jeweils durch direkte zueinander parallele Verbindungsnähte 15 der beiden Trennwände 4 verbunden. Es sind daher zwei vorfertigbare Einsatzkörper 29 gegeben, von denen jeder an der Innenseite einer der beiden Folien 2', 2" entlang seines Randes 24 befestigt wird. Auch hier sind die Verbindungsnähte 15 der beiden Einsatzkörper 29 um die halbe Kammernbreite versetzt, sodaß sich beim Evakuieren des Innenraumes jeder Scheitel 14 sich in den Bereich der gegenüberliegenden Verbindungsnaht 15 schmiegt.

Die luftdurchlässigen Trennwände 4 und die schräggestellten Stege 8 bzw. die Einlage 7 sind bevorzugt aus einem Gewebe aus Polypropylenfasern hergestellt. Die Verbindungen 15 zwischen den Geweben sind daher bevorzugt genäht und die Verbindungen 9 mit den Folien 2', 2" geschweißt.

Eine weitere Ausführung eines Folienelementes kann für die Stabilisierung und Stützung des Nackenbereiches allein verwendet werden, und weist dann etwa die in Fig. 1 oder 7 gezeigte Form, jedoch mit wesentlich kürzerem Rumpfteil 22 auf. Diese Ausführung stellt daher eine Halsmanschette dar, die unabhängig von der Körpergröße einsetzbar ist.

## Patentansprüche

1. Einrichtung zur Stützung und Stabilisierung eines verletzten Körperteils, mit einem flexiblen, um den Körperteil festlegbaren Folienelement (1), dessen beiden Folien (2', 2") einen luftdichten, evakuierbaren Innenraum einschließen, in dem lose Partikel (6) enthaltende langgestreckte Kammern (5) vorgesehen sind, **dadurch gekennzeichnet, daß** der Innenraum durch mindestens zwei luftdurchlässige, flexible Trennwände (4) in zumindest drei Fächer (3) unterteilt ist, und die Kammern (5) in zumindest einem der drei Fächer (3) ausgebildet sind.

2. Einrichtung nach Anspruch 1, **dadurch gekennzeichnet, daß** die Kammern (5) im mittleren von drei Fächern (3) vorgesehen und durch schräggestellte Stege (8) zwischen den beiden flexiblen Trennwänden (4) gebildet sind.

3. Einrichtung nach Anspruch 2, **dadurch gekennzeichnet, daß** die schräggestellten Stege (8) durch eine in Wellen gelegte Einlage (7) gebildet sind, die jeweils in den Umkehrbereichen mit den beiden flexiblen Trennwänden (4) verbunden sind.

4. Einrichtung nach Anspruch 1, **dadurch gekennzeichnet, daß** der Innenraum durch vier flexible Trennwände (4) in fünf Fächer (3) unterteilt ist, und die Kammern (5) im zweiten und im vierten Fach (3) vorgesehen und durch mehrere parallele Verbindungsnähte (9) zwischen jeweils zwei flexiblen Trennwänden (4) gebildet sind, wobei die gegenüberliegenden Verbindungsnähte (15) jeweils um die halbe Kammerbreite zueinander parallel versetzt sind.

5. Einrichtung nach Anspruch 1, **dadurch gekennzeichnet, daß** die Kammern (5) in den beiden äußeren von drei Fächern (3) vorgesehen und durch mehrere parallele Verbindungsnähte (9) zwischen jeweils einer Folie (2', 2") des Folienelementes (1) und der benachbarten flexiblen Trennwand (4) gebildet sind, wobei die gegenüber liegenden Verbindungsnähte (9) jeweils um die halbe Kammerbreite zueinander parallel versetzt sind.

6. Einrichtung nach einem der Ansprüche 1 bis 4, **dadurch gekennzeichnet, daß** je zwei luftdurchlässige, flexible Trennwände (4) entlang ihrer Ränder (24) miteinander zu einem die Partikel (6) enthaltendem Einsatzkörper (29) verbunden sind, der zwischen den Rändern (23) der beiden Folien (2', 2") angeordnet und mit diesen verbunden ist.

7. Einrichtung nach einem der Ansprüche 1 bis 5, **dadurch gekennzeichnet, daß** die flexiblen Trennwände (4) aus Gewebebahnen hergestellt sind.

8. Einrichtung nach einem der Ansprüche 1 bis 7, **dadurch gekennzeichnet, daß** sie als Halsmanschette ausgebildet ist.

9. Einrichtung nach einem der Ansprüche 1 bis 7 zur Stützung und Stabilisierung des Oberkörpers eines Verletzten, mit einem flexiblen, an den Oberkörper anlegbaren Folienelement (1), das einen Rumpfteil (22) und einen Kopfteil (20) aufweist, wobei der Kopfteil (20) beidseitig mit einer Schulterabstützung (25) versehen ist, und dessen beiden Folien (2', 2") einen luftdichten, evakuierbaren Innenraum einschließen, in dem lose Partikel (6) enthalten sind, **dadurch gekennzeichnet, daß** die Partikel (6) in Kammern (5) vorgesehen sind, deren Breite im Kopfteil (20) kleiner ist als im Rumpfteil (22).

10. Einrichtung nach Anspruch 9, **dadurch gekennzeichnet, daß** jede Schulterabstützung (25) mit Verbindungsgurten (26) zur Vorderseite des Rumpfteiles (22) versehen ist.

11. Einrichtung nach einem der Ansprüche 1 bis 10, mit Befestigungsgurten (11), die in Umfangsrichtung des jeweiligen Körperteiles schließbar sind, **dadurch gekennzeichnet, daß** die Befestigungsgurten (11) zumindest teilweise zwischen der vom verletzten Körperteil abgewandten Folie (2") und einem zusätzlichen Folienstück (10) angeordnet sind.

12. Einrichtung nach einem der Ansprüche 1 bis 11, **dadurch gekennzeichnet, daß** die Kammern (5) einander teilweise so überlagem, daß bei evakuiertem Innenraum Partikel (6) benachbarter Kammern (5) einander zu einer im wesentlichen gleichmäßigen Partikelschicht ergänzen.

## Claims

1. Apparatus for supporting and stabilising an injured part of the body, comprising a flexible sheet element (1) which can be fixed around the part of the body and the two sheets (2', 2") of which enclose an air-tight evacuatable internal space in which are provided elongate chambers (5) containing loose particles (6), **characterised in that** the internal space is subdivided into at least three compartments (3) by at least two air-permeable flexible dividing walls (4) and the chambers (5) are provided in at least one of the three compartments (3).

2. Apparatus according to claim 1 **characterised in that** the chambers (5) are provided in the central one of three compartments (3) and are formed by inclined webs (8) between the two flexible dividing walls (4).

3. Apparatus according to claim 2 **characterised in that** the inclined webs (8) are formed by an insert (7) which is laid in undulations and which are each connected in the reversal regions to the two flexible dividing walls (4).

4. Apparatus according to claim 1 **characterised in that** the internal space is divided into five compartments (3) by four flexible dividing walls (4) and the chambers (5) are provided in the second and fourth compartments (3) and are formed by a plurality of parallel connecting seams (9) between each two flexible dividing walls (4), wherein the mutually opposite connecting seams (15) are each displaced in parallel relationship with each other by half the chamber width.

5. Apparatus according to claim 1 **characterised in that** the chambers (5) are provided in the outer two of three compartments (3) and are formed by a plurality of parallel connecting seams (9) between a respective sheet (2', 2") of the sheet element (1) and the adjacent flexible dividing wall (4), wherein the mutually opposite connecting seams (9) are each displaced in parallel relationship with respect to each other by half the chamber width.

6. Apparatus according to one of claims 1 to 4 **characterised in that** two respective air-permeable flexible dividing walls (4) are connected together along their edges (24) to form an insert body (29) which contains the particles (6) and which is arranged between the edges (23) of the two sheets (2', 2") and is connected to them.

7. Apparatus according to one of claims 1 to 5 **characterised in that** the flexible dividing walls (4) are produced from fabric webs.

8. Apparatus according to one of claims 1 to 7 **characterised in that** it is in the form of a neck cuff.

9. Apparatus according to one of claims 1 to 7 for supporting and stabilising the upper body of an injured person comprising a flexible sheet element (1) which can be applied to the upper body and which has a trunk part (22) and a head part (20), wherein the head part (20) is provided on both sides with a shoulder support (25) and the two sheets (2', 2") of which enclose an air-tight evacuatable internal space in which are contained loose particles (6), **characterised in that** the particles (6) are provided in chambers (5), the width of which is smaller in the head part (20) than in the trunk part (22).

10. Apparatus according to claim 9 **characterised in that** each shoulder support (25) is provided with connecting straps (26) for connection to the front side of the trunk part (22).

11. Apparatus according to one of claims 1 to 10 with fixing straps (11) which can be closed in the peripheral direction of the respective part of the body, **characterised in that** the fixing straps (11) are at least partially arranged between the sheet (2") which faces away from the injured part of the body and an additional sheet portion (10).

12. Apparatus according to one of claims 1 to 11 **characterised in that** the chambers (5) are partially in mutually superposed relationship such that when the internal space is evacuated particles (6) of adjacent chambers (5) supplement each other to form a substantially uniform particle layer.

## Revendications

1. Dispositif servant à soutenir et à stabiliser une partie corporelle blessée, comprenant un élément souple en feuilles (1) et pouvant être fixé autour de la partie corporelle, et dont les deux feuilles (2', 2") définissent un espace intérieur étanche à l'air, dans lequel le vide peut être fait, et dans lequel il est prévu des chambres (5) allongées contenant des particules libres (6), **caractérisé en ce que** l'espace intérieur est subdivisé en au moins trois compartiments (3) par au moins deux parois de séparation (4) souples et perméables à l'air, et **en ce que** les chambres (5) sont formées dans au moins l'un des trois compartiments (3).

2. Dispositif selon la revendication 1, **caractérisé en ce que** les chambres (5) sont prévues dans le compartiment médian des trois compartiments (3) et sont définies par des barrettes (8) disposées obliquement entre les deux parois souples de séparation (4).

3. Dispositif selon la revendication 2, **caractérisé en ce que** les barrettes (8) disposées obliquement sont constituées par un élément intercalaire (7) placé de manière ondulée et dont les zones de renversement sont chaque fois fixées aux deux parois souples de séparation (4).

4. Dispositif selon la revendication 1, **caractérisé en ce que** l'espace intérieur est subdivisé par quatre parois souples de séparation (4) en cinq compartiments (3), et **en ce que** les chambres (5) sont prévues dans les deuxième et quatrième compartiments (3) et sont formées par plusieurs soudures parallèles de jonction (9) entre chaque fois deux parois souples de séparation (4), les soudures de jonction (15) opposées étant chaque fois décalées parallèlement les unes aux autres de la moitié de la largeur de chambre.

5. Dispositif selon la revendication 1, **caractérisé en ce que** les chambres (5) sont prévues dans les deux compartiments externes de trois compartiments (3), et sont formées par plusieurs soudures parallèles de jonction (9) entre chaque fois une feuille (2', 2") de l'élément en feuilles (1) et la paroi souple de séparation (4) voisine, les soudures de jonction (9) opposées étant chaque fois décalées parallèlement les unes aux autres de la moitié de la largeur de chambre.

6. Dispositif selon l'une des revendications 1 à 4, **caractérisé en ce que** chaque fois deux parois de séparation (4) souples et perméables à l'air sont reliées l'une à l'autre le long de leurs bords (24) pour constituer un corps inséré (29) contenant les particules (6) et qui est disposé entre les bords (23) des deux feuilles (2', 2") et relié à celles-ci.

7. Dispositif selon l'une des revendications 1 à 5, **caractérisé en ce que** les parois souples de séparation (4) sont fabriquées à partir de bandes de tissu.

8. Dispositif selon l'une des revendications 1 à 7, **caractérisé en ce qu'**il est réalisé sous la forme d'une minerve.

9. Dispositif selon l'une des revendications 1 à 7, servant à soutenir et à stabiliser la partie corporelle supérieure d'un blessé, le dispositif comprenant un élément en feuilles (1) pouvant être appliqué contre la partie corporelle supérieure et comprenant une partie tronc (22) et une partie tête (20), la partie tête (20) étant pourvue des deux côtés d'un soutien d'épaule (25), et dont les deux feuilles (2', 2") définissent un espace intérieur étanche à l'air, dans lequel le vide peut être fait, et dans lequel sont contenues des particules libres (6), **caractérisé en ce que** les particules (6) sont prévues dans des chambres (5), dont la largeur est plus petite dans la partie tête (20) que dans la partie tronc (22).

10. Dispositif selon la revendication 9, **caractérisé en ce que** chaque soutien d'épaule (25) est pourvu de sangles de liaison (26) avec le côté avant de la partie tronc (22).

11. Dispositif selon l'une des revendications 1 à 10, comprenant des sangles de fixation (11), qui peuvent être serrées autour de la partie corporelle correspondante, **caractérisé en ce que** les sangles de fixation (11) sont disposées au moins en partie entre la feuille (2"), dirigée à l'opposé de la partie corporelle blessée, et un morceau de feuille (10) supplémentaire.

12. Dispositif selon l'une des revendications 1 à 11, **caractérisé en ce que** les chambres (5) sont mutuellement à superposition partielle, de telle sorte que, lorsque le vide est fait dans l'espace intérieur, des particules (6) de chambres (5) voisines se complètent pour former une couche de particules, qui est sensiblement régulière.
